# EUROPEAN PATENT APPLICATION

(11) **EP 3 763 675 A1**
(43) Date of publication of application: **13.01.2021**
(21) Application number: 19185982.6
(22) Date of filing: 12.07.2019
(51) Int. Cl.: C01F 11/06, C10G 2/00, C07C 1/12

(54) **SYNTHETIC HYDROCARBON GAS PRODUCTION SYSTEM AND METHOD**

(71) Applicant: Hymeth ApS, 2000 Frederiksberg C (DK)
(72) Inventor: Bishwas, Sumon, DK-2660 Brøndby Strand, Copenhagen (DK)
(74) Representative: Kransell & Wennborg KB

(57) **Abstract**

A synthetic hydrocarbon gas production system (1) comprising: a hydrogen gas source (3), a carbon dioxide source (5) comprising: a thermal decomposition reaction chamber (5a) configured to receive calcium carbonate (6), and a heating device (5b) configured to heat the thermal decomposition reaction chamber to a thermal decomposition temperature of calcium carbonate to release carbon dioxide from calcium carbonate; and a hydrocarbon gas reactor (7), wherein the carbon dioxide source (5; 5") is configured to supply carbon dioxide from the thermal decomposition reaction chamber (5a) to the hydrocarbon gas reactor (7) and the hydrogen gas source (3: 3') is configured to supply hydrogen gas to the hydrocarbon gas reactor (7) to react the carbon dioxide (15) with the hydrogen gas (13) to obtain a synthetic hydrocarbon gas (17).

## Description

### TECHNICAL FIELD

The present disclosure relates to a system and method for producing a synthetic hydrocarbon gas.

### BACKGROUND

Electrolysis is the process of converting water to hydrogen gas and oxygen gas by means of electricity. The hydrogen gas thus obtained can be reacted with carbon to obtain methane gas. WO2019/057764 discloses a system of this type. It is also known to use carbon dioxide to mix with the hydrogen gas.

The use of solid carbon or carbon dioxide may in general not be suitable to use, especially in a large-scale electrolysis system, from an environmental perspective as well as from a cost perspective when producing methane. For example, burning the methane gas or hydrocarbon fuel produced from methane gas, adds more carbon dioxide into the atmosphere.

### SUMMARY

If carbon dioxide is produced from fossil fuel/biomass or other similar sources and combined with hydrogen gas to make synthetic hydrocarbon gas, more carbon dioxide is added into the atmosphere. The process will hence not be environmentally friendly.

In view of the above, a general object of the present disclosure is to solve or at least reduce the problems of the prior art.

There is hence according to a first aspect of the present disclosure provided a synthetic hydrocarbon gas production system comprising: a hydrogen gas source, a carbon dioxide source comprising: a thermal decomposition reaction chamber configured to receive calcium carbonate, and a heating device configured to heat the thermal decomposition reaction chamber to a thermal decomposition temperature of calcium carbonate to release carbon dioxide from calcium carbonate; and a hydrocarbon gas reactor, wherein the carbon dioxide source is configured to supply carbon dioxide from the thermal decomposition reaction chamber to the hydrocarbon gas reactor and the hydrogen gas source is configured to supply hydrogen gas to the hydrocarbon gas reactor to react the carbon dioxide with the hydrogen gas to obtain a synthetic hydrocarbon gas.

Carbon dioxide for reacting with the hydrogen gas may thereby be obtained in an environmentally friendly manner. This may be especially beneficial in large-scale synthetic hydrocarbon gas production.

The thermal decomposition reaction in the thermal decomposition reaction chamber leaves lime, i.e. calcium oxide as a by-product. The calcium oxide can absorb carbon dioxide from the air when in contact with ambient air. This makes the cycle carbon neutral. Hence, by using the present method to produce synthetic hydrocarbon gas or fuel from the synthetic hydrocarbon gas, less carbon dioxide will be produced using the gas/fuel, and by producing lime, it will absorb the same amount of carbon dioxide from the atmosphere if released.

The calcium oxide may be used for cement production or similar industrial applications. In this case, the process of producing the synthetic hydrocarbon gas will become carbon negative. Normally in a cement factory, fossil fuel is used to produce lime from limestone. The fossil fuel as well as the calcination of limestone releases carbon dioxide into the air. Using green lime, the carbon dioxide emission can be reduced from burning fuel as well as from burning limestone. Hence, using the by-product lime, i.e. green lime, there will be a negative carbon footprint.

Alternatively, the by-product lime could be released into the sea to make acidic sea water more alkaline. Hence, the pH value of water will reverse from acidic to alkaline again. This means that sea water can then absorb more carbon dioxide from the air as a natural process while bringing the pH value back to normal, thereby helping the marine environment, especially sea creatures that only thrive in slightly alkaline water.

The calcium carbonate may be contained in limestone. The thermal decomposition reaction chamber may hence be configured to receive limestone.

Limestone absorbs carbon dioxide from the atmosphere very well. 10 kilograms of limestone may form about 9 kilograms of carbon dioxide in a thermal decomposition reaction. Limestone is hence a very good source of carbon dioxide.

The carbon dioxide source may be a calcination device.

The carbon dioxide may be carbon dioxide gas. The hydrocarbon gas reactor may be configured to react carbon dioxide gas with hydrogen gas.

The hydrogen gas source may be a green hydrogen gas source or non-fossil fuel produced hydrogen gas source.

The hydrocarbon gas reactor may be configured to operate under high pressure and temperature.

The heating device may be configured to be heated electrically for example by using one or more renewable energy sources such as solar cells or wind turbines.

According to one embodiment the thermal decomposition reactor chamber is a vacuum chamber.

According to one embodiment the hydrogen gas source comprises an electrolyser configured to produce hydrogen gas.

Using hydrogen gas and carbon dioxide from green and sustainable sources, i.e. hydrogen gas from electrolysis and carbon dioxide from the thermal decomposition reaction, the synthetic hydrocarbon gas will be environmentally friendly. Further, the cost of producing green synthetic hydrocarbon gas can be drastically reduced as currently existing ideas/solutions require large amount of energy for production.

By means of the present synthetic hydrocarbon gas production system it is possible to take more carbon dioxide from the air than the produced synthetic hydrocarbon gas will release back into the atmosphere after combustion. Further, the cost of producing the synthetic hydrocarbon gas will drastically decrease.

The electrolyser may be a large-scale electrolyser. A large-scale electrolyser may be an electrolyser that consumes 500 kilowatt (kW) or more, such as 750 kW or more, such as 1 MW or more, to produce hydrogen gas and oxygen gas.

According to one embodiment the electrolyser is a high-pressure electrolyser. The synthetic hydrocarbon gas production system may hence comprise one or more pumps configured to pump water into the electrolyser to pressurise the water inside the electrolyser.

The synthetic hydrocarbon gas production system may comprise a pressure compensator to compensate for pressure differences between an oxygen gas channel and a hydrogen gas channel in the electrolyser.

According to one embodiment the hydrogen gas source comprises a hydrogen gas pressure vessel configured to supply hydrogen gas to the hydrocarbon gas reactor.

According to one embodiment the electrolyser is configured to discharge hydrogen gas to the hydrogen gas pressure vessel and the hydrogen gas pressure vessel is configured to supply hydrogen gas collected from the electrolyser to the hydrogen gas pipe.

According to one embodiment the carbon dioxide source comprises a carbon dioxide pressure vessel configured to collect carbon dioxide from the thermal decomposition reactor chamber and configured to supply carbon dioxide collected from the thermal decomposition reactor chamber to the hydrocarbon gas reactor.

One embodiment comprises a pressurising device configured to pressurise carbon dioxide from the carbon dioxide source upstream of the hydrocarbon gas reactor.

One embodiment comprises a filter configured to filter carbon dioxide upstream of the hydrocarbon gas reactor.

There is according to a second aspect of the present disclosure provided a method of producing a synthetic hydrocarbon gas, wherein the method comprises: a) thermally decomposing calcium carbonate in a thermal decomposition reaction chamber by applying heat to release carbon dioxide from the calcium carbonate, and b) reacting the carbon dioxide with hydrogen gas in a hydrocarbon gas reactor to obtain the synthetic hydrocarbon gas.

The hydrogen gas may have been produced using a non-fossil fuel production method.

One embodiment comprises directing the carbon dioxide and the hydrogen gas to the hydrocarbon gas reactor before step b).

One embodiment comprises generating the hydrogen gas in an electrolyser and directing the hydrogen gas from the electrolyser to the hydrocarbon gas reactor.

One embodiment comprises directing the hydrogen gas from a hydrogen gas pressure vessel to the hydrocarbon gas reactor.

According to one embodiment the thermal decomposition reaction chamber is a vacuum chamber.

According to one embodiment the calcium carbonate is contained in limestone arranged in the thermal decomposition reaction chamber.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated.

### BRIEF DESCRIPTION OF THE DRAWINGS

Examples of the inventive concept will now be described, by way of example, with reference to the accompanying drawings, in which:
Fig. 1a shows a diagram of an example of a synthetic hydrocarbon gas production system;
Fig. 1b shows a diagram of another example of a synthetic hydrocarbon gas production system;
Fig. 1c shows a diagram of another example of a synthetic hydrocarbon gas production system; and
Fig. 2 is a flowchart of a method of producing a synthetic hydrocarbon gas using a synthetic hydrocarbon gas production system.

### DETAILED DESCRIPTION

The invention will now be described more fully hereinafter with reference to the accompanying drawings, in which certain embodiments of the invention are shown. This invention may, however, be embodied in many different forms and should not be construed as limited to the embodiments set forth herein; rather, these embodiments are provided by way of example so that this disclosure will be thorough and complete, and will fully convey the scope of the invention to those skilled in the art. Like numbers refer to like elements throughout the description.

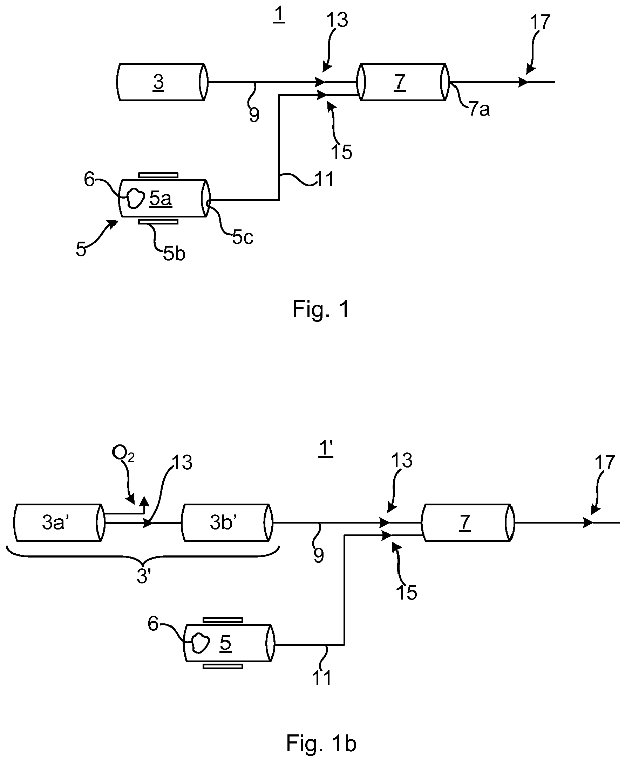

Fig. 1a shows an example of a synthetic hydrocarbon gas production system 1. The synthetic hydrocarbon gas production system 1 comprises a hydrogen gas source 3, a carbon dioxide source 5, and a hydrocarbon gas reactor 7. The hydrogen gas source 3 is configured to supply hydrogen gas 13 to the hydrocarbon gas reactor 7. The carbon dioxide source 5 is configured to supply carbon dioxide 15 to the hydrocarbon gas reactor 7. The carbon dioxide 15 and the hydrogen gas 13 are reacted in the hydrocarbon gas reactor 7 to obtain a stream of synthetic hydrocarbon gas 17.

The carbon dioxide source 5 comprises a thermal decomposition reaction chamber 5a. The thermal decomposition reaction chamber 5a may be a vacuum chamber. The thermal decomposition reaction chamber 5a is configured to receive calcium carbonate 6. The calcium carbonate 6 may be contained in limestone. The thermal decomposition reaction chamber 5a may hence be configured to receive limestone.

The carbon dioxide source 5 comprises a heating device 5b. The heating device 5b is configured to heat the thermal decomposition reaction chamber 5a. The heating device 5b may be configured to heat the calcium carbonate 6 or limestone arranged inside the thermal decomposition reaction chamber 5a to a temperature equal to or above a thermal decomposition temperature of calcium carbonate 6 or limestone.

The heating device 5b may for example be an electrical heating device 5b. The synthetic hydrocarbon gas production system 1 may comprise one or more renewable energy sources such as solar cells and/or wind turbines and/or wave energy converters configured to power the electrical heating device 5b. The heating device 5b could alternatively be a fuel-based heating device.

The synthetic hydrocarbon gas production system 1 comprises a hydrogen gas pipe 9 and a carbon dioxide gas pipe 11.

The thermal decomposition reaction chamber 5a comprises a carbon dioxide outlet 5c. According to the example, the carbon dioxide gas pipe 11 is connected to the carbon dioxide outlet 5c and to the hydrocarbon gas reactor 7. Carbon dioxide 15 is directed from the thermal decomposition reaction chamber 5a to the hydrocarbon gas reactor 7 by means of the carbon dioxide pipe 11.

The hydrogen gas pipe 9 is connected to the hydrogen gas source 3. Hydrogen gas 13 is directed from the hydrogen gas source 3 to the hydrocarbon gas reactor 7 by means of the hydrogen gas pipe 9. The hydrogen gas 13 and the carbon dioxide 15 are reacted in the hydrocarbon gas reactor 7 to form the synthetic hydrocarbon gas 17. The synthetic hydrocarbon gas 17 is methane gas.

The hydrocarbon gas reactor 7 has a synthetic hydrocarbon gas outlet 7a configured to discharge the stream of synthetic hydrocarbon gas 17.

In the example in Fig. 1a, the hydrogen gas source 3 comprises only one of an electrolyser and a hydrogen gas pressure vessel. Fig. 1b shows an example of a synthetic hydrocarbon gas production system 1' in which the hydrogen gas source 3' comprises an electrolyser 3a' and a hydrogen gas pressure vessel 3b'. The electrolyser 3a' produces hydrogen gas 13 and oxygen gas O2. The hydrogen gas 13 is directed to flow to the hydrogen gas pressure vessel 3b' for temporary storage of hydrogen gas. The hydrogen gas pressure vessel 3b' hence collects the hydrogen gas 13 generated by the electrolyser 3a'. The hydrogen gas pressure vessel 3b' is connected to the hydrocarbon gas reactor 7. The hydrogen gas pressure vessel 3b' is hence arranged downstream of the electrolyser 3a' but upstream of the hydrocarbon gas reactor 7. The carbon dioxide 15 is directed to flow from the carbon dioxide source 5 to the hydrocarbon gas reactor 7 to react with the hydrogen gas 13 from the hydrogen gas pressure vessel 3b'.

The hydrogen gas could alternatively be provided to the hydrocarbon gas reactor 7 from both the electrolyser and the hydrogen gas pressure vessel simultaneously.

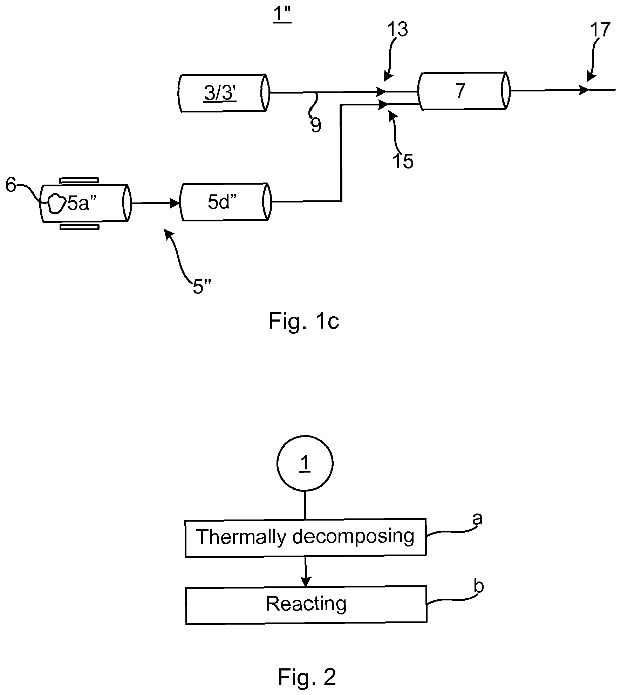

Fig. 1c shows another example of a hydrocarbon gas production system 1". In this example, the carbon dioxide source 5" further comprises a carbon dioxide pressure vessel 5d" for temporary storage of carbon dioxide generated by the thermal decomposition reactor chamber 5a". The thermal decomposition reactor chamber 5a" is connected to the carbon dioxide pressure vessel 5d". The thermal decomposition reactor chamber 5a" is configured to discharge the carbon dioxide to the carbon dioxide pressure vessel 5d". The carbon dioxide pressure vessel 5d" is configured to be connected to the hydrocarbon gas reactor 7. The carbon dioxide pressure vessel 5d" is hence arranged downstream of the thermal decomposition reactor chamber 5a" but upstream of the hydrocarbon gas reactor 7. In this example, the hydrogen gas source 3 may comprise only one of the electrolyser and the hydrogen gas pressure vessel, or both.

The hydrocarbon gas production system 1, 1', 1" may comprise a pressurising device configured to pressurise the carbon dioxide discharged from the thermal decomposition reactor chamber 5a. The pressurising device may for example be a compressor.

The hydrocarbon gas production system 1, 1', 1" may comprise a filter configured to filter carbon dioxide discharged from the thermal decomposition reactor chamber 5a to obtain a cleaner carbon dioxide. The filter may be arranged to filter the carbon dioxide before the carbon dioxide is directed into the hydrocarbon gas reactor 7. The filter is hence arranged downstream of the thermal decomposition reactor chamber 5a" and upstream of the hydrocarbon gas reactor 7.

Fig. 2 shows a method of producing a synthetic hydrocarbon gas by means of the synthetic hydrocarbon gas production system 1, 1', 1".

The calcium carbonate 6, for example in the form of limestone, is first placed in the thermal decomposition reactor chamber 5a. A vacuum or lower pressure than the ambient pressure is then created in the thermal decomposition reactor chamber 5a.

In a step a) the calcium carbonate 6 is heated to a temperature corresponding at least to a thermal decomposition temperature of calcium carbonate. This temperature may be above 600 °C, such as above 800 °C, for example at least 840 °C. The calcium carbonate 6 in the thermal decomposition reactor chamber 5a thereby releases carbon dioxide in a thermal decomposition reaction or calcination. Calcium oxide or quicklime is formed inside the thermal decomposition reactor chamber 5a as a by-product of the thermal decomposition reaction.

The carbon dioxide formed in the thermal decomposition reaction is discharged from or exits the thermal decomposition reactor chamber 5a through the carbon dioxide outlet 5c and flows through the carbon dioxide gas pipe 11 into the hydrocarbon gas reactor 7. Depending on the realisation of the synthetic hydrocarbon gas production system 1, 1', 1", carbon dioxide may or may not flow from the thermal decomposition reactor chamber 5a via the optional carbon dioxide pressure vessel 5d".

Hydrogen gas 13 from the hydrogen source 3 flows to the hydrocarbon gas reactor 7 and is mixed with the carbon dioxide 15 from the carbon dioxide source 5. Hence in a step b) the carbon dioxide 15 is reacted with the hydrogen gas 13 in the hydrocarbon gas reactor 7. A stream of synthetic hydrocarbon gas 17 and water is thereby obtained.

The synthetic hydrocarbon gas 17, i.e. methane gas, may be used as is or it may for example be processed to obtain liquid fuel such as methanol, diesel or petrol.

When all the calcium carbonate 6 has been reacted in the thermal decomposition reaction, about 90% of the calcium carbonate 6 has turned to carbon dioxide and the rest has turned into solid calcium oxide. The by-product calcium oxide is at this point arranged in the thermal decomposition chamber 5a and can be removed when all the calcium carbonate 6 has been reacted. The calcium oxide may for example be used to manufacture cement or released into the sea to counteract acidity of the sea. Hereto both the carbon dioxide and the only by-product calcium oxide may be fully utilised.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A synthetic hydrocarbon gas production system (1; 1'; 1") comprising:
a hydrogen gas source (3; 3'),
a carbon dioxide source (5; 5") comprising:
a thermal decomposition reaction chamber (5a; 5a") configured to receive calcium carbonate (6), and
a heating device (5b) configured to heat the thermal decomposition reaction chamber to a thermal decomposition temperature of calcium carbonate to release carbon dioxide (15) from calcium carbonate (6); and
a hydrocarbon gas reactor (7),
wherein the carbon dioxide source (5; 5") is configured to supply carbon dioxide from the thermal decomposition reaction chamber (5a; 5a") to the hydrocarbon gas reactor (7) and the hydrogen gas source (3: 3') is configured to supply hydrogen gas to the hydrocarbon gas reactor (7) to react the carbon dioxide (15) with the hydrogen gas (13) to obtain a synthetic hydrocarbon gas (17).

2. The synthetic hydrocarbon gas production system (1; 1'; 1") as claimed in claim 1, wherein the thermal decomposition reactor chamber (7) is a vacuum chamber.

3. The synthetic hydrocarbon gas production system (1; 1'; 1") as claimed in claim 1 or 2, wherein the hydrogen gas source (3; 3') comprises an electrolyser (3a') configured to produce hydrogen gas.

4. The synthetic hydrocarbon gas production system (1; 1'; 1") as claimed in claim 4, wherein the electrolyser (3a') is a high-pressure electrolyser.

5. The synthetic hydrocarbon gas production system (1; 1'; 1") as claimed in any of the preceding claims, wherein the hydrogen gas source (3; 3') comprises a hydrogen gas pressure vessel (3b') configured to supply hydrogen gas to the hydrocarbon gas reactor (7).

6. The synthetic hydrocarbon gas production system (1") as claimed in any of the preceding claims, wherein the carbon dioxide source (5") comprises a carbon dioxide pressure vessel (5d") configured to collect carbon dioxide from the thermal decomposition reactor chamber (5a") and configured to supply carbon dioxide collected from the thermal decomposition reactor chamber (5a") to the hydrocarbon gas reactor (7).

7. The synthetic hydrocarbon gas production system (1; 1'; 1") as claimed in any of the preceding claims, comprising a pressurising device configured to pressurise carbon dioxide from the carbon dioxide source upstream of the hydrocarbon gas reactor (7).

8. The synthetic hydrocarbon gas production system (1; 1'; 1") as claimed in any of the preceding claims, comprising a filter configured to filter carbon dioxide upstream of the hydrocarbon gas reactor (7).

9. A method of producing a synthetic hydrocarbon gas (17), wherein the method comprises:
a) thermally decomposing calcium carbonate (6) in a thermal decomposition reaction chamber (5a; 5a") by applying heat to release carbon dioxide (15) from the calcium carbonate (6), and
b) reacting the carbon dioxide (15) with hydrogen gas (13) in a hydrocarbon gas reactor (7) to obtain the synthetic hydrocarbon gas (17).

10. The method as claimed in claim 9, comprising directing the carbon dioxide (15) and the hydrogen gas (13) to the hydrocarbon gas reactor (7) before step b).

11. The method as claimed in claim 9 or 10, comprising generating the hydrogen gas (13) in an electrolyser (3a') and directing the hydrogen gas (13) from the electrolyser (3a') to the hydrocarbon gas reactor (7).

12. The method as claimed in claim 9 or 10, comprising directing the hydrogen gas (13) from a hydrogen gas pressure vessel (3b') to the hydrocarbon gas reactor (7).

13. The method as claimed in any of claims 9-12, wherein the thermal decomposition reaction chamber (7) is a vacuum chamber.

14. The method as claimed in any of claims 9-13, wherein the calcium carbonate (6) is contained in limestone arranged in the thermal decomposition reaction chamber (5a; 5a").
